# EUROPEAN PATENT APPLICATION

(11) **EP 1 197 194 A1**
(43) Date of publication of application: **17.04.2002**
(21) Application number: 00111979.1
(22) Date of filing: 10.10.2000
(51) Int. Cl.: A61F 5/443

(54) **Expanding applicator for a human waste collection bag**

(71) Applicant: The Procter & Gamble Company, Cincinnati, Ohio 45202 (US)
(72) Inventor: Evangelista, Olindo, 66023 Francavilla al Mare, Chieti (IT); Spinelli, Ivana, 65100 Pescara (IT); Sullivan, Ann Marie, Waltham, MA 02451 (US); Sutton, Thomas, 20127 Milano (IT)
(74) Representative: Hirsch, Uwe Thomas

(57) **Abstract**

The present invention relates to human waste collection bags for babies, children or adults to be attached to a wearer. More particularly it relates to an applicator for such collection bags, which can be used to place the collection bag in the perianal or uro-genital area of a wearer and allows convenient and accurate placement. Claimed and described is an applicator (40) comprising at least one handling portion (42) and at least one contact area (44); which upon application of force between said at least one handling portion (42) and the at least one contact area (44) expands in at least one direction of expansion.

## Description

### Field of the invention

The present invention relates to human waste collection bags for babies, children or adults to be attached to a wearer. More particularly it relates to an applicator for such a collection bag, which can be used to place the collection bag in the perianal or uro-genital area of a wearer and allows convenient and accurate placement.

### Background of the invention

Human waste collection bags are known articles of manufacture that are designed to be worn principally by incontinence sufferers and in particular by bedridden patients. Such human waste collection bags are attached to the perianal area or uro-genital area of the wearer and are intended to entrap and immediately contain faecal material, urine and other bodily discharges.

Faecal collection bags as they are mostly known today are constituted of a relatively long and narrow tube, at one extremity of which is positioned the aperture and the attachment device, which can be adhesive. Such bags are disclosed in, e. g. US 3,577,989.

A problem naturally associated with these devices is their attachment to the human body. The approach which is mostly used in the field is to provide the device with an adhesive flange, which will stick to the perianal or uro-genital area.

US 3,522,807 and US 3,734,096 disclose faecal receptacles having an adhesive flange surrounding the aperture in the device, for attachment to the body of the patient in nursing or medical applications; said flange contains a plurality of tabs extending outwardly from the aperture and said tabs are covered with adhesive in the same manner as the rest of the flange and thus are designed to serve as adhering aids, and must be covered by a release means before use of the receptacles.

In GB-A-2,116,849, it was attempted to provide an adhesive faecal incontinence device which, among other properties, was easier to put in place on the patient. The solution brought up by GB-A-2,116,849 is, however, quite complex, involving individually removable sections of the release layer covering the adhesive layer on the flange surrounding the aperture, said sections having to be removed in a predetermined sequence in order to ensure optimum adherence.

Besides and in connection with optimum adherence, the correct placement of the device is also a key issue in the field of human waste collection bags. Total or substantial misplacement of the device will lead to a severe misfunctioning, in particular incomplete collection of faeces or urine and leakage. For example, if the aperture of a faecal collection bag is not sufficiently in registry with the anal opening, substantial pressure, in particular on the flanges of the device, can build up in the defecation process. Such substantial pressure can lead to the detachment of the adhesively secured device, obviously entailing the most unwanted consequences.

If the misplacement of the device is recognised before use, the placement of the device is normally corrected, typically by the carer. The necessary detachment and reattachment of the device causes an additional stress on the affected areas of skin of the wearer. Many wearers, who make use of faecal collection bags have a sensitive skin due to their age, whether very old or very young, and furthermore sometimes also suffer from skin irritations. Proper placement of the device in the first place is therefore highly desirable.

A particularly relevant issue in relation to the placement of human waste collection bag provided with an adhesive flange is premature sticking. Typically a release paper which covers the flange is removed as an early step in the application of a human waste collection bag. Contact of the thereby exposed adhesive of the flange with any portion of the skin of the caretaker or wearer will leave to adhesion o at least a portion of the flange to the contacted portion of the skin.

In case of a faecal collection bag premature sticking often occurs with portions of the buttocks more remote from the anal opening than the portions of the buttocks intended to make the adhesive contact with the flange.

In case of a urine collection bag premature sticking may occur with the thighs of the wearer. Depending on the individual anatomy of a wearer either the buttocks or the thighs may not only engage in premature sticking but may irrespectively make it difficult to access the uro-genital or perineal/coccygeal area, respectively, onto which a human waste collection bag is to be placed.

In Kokai Patent Application No. HE18 (1996) 117 261, an external accessory is described to assist in the placement of the adhesive part of the disclosed diaper onto the wearer. Such a tool may help in the placement of such an incontinence product when compared to the placement without any aide. However, the successful use of such a tool will require training, in particular if the tool is not specifically designed for its purpose. A further problem with such a tool is that a caretaker, for example, when dealing with a bedridden patient, may have only one hand available for the application of the device. Further the tool will not help in avoiding premature sticking.

A recent patent application WO 00/00126 discloses an applicator for a faecal collection bag, which is a very helpful device and due to its slim shape in a preferred embodiment allows access to the perianal area without inducing a high risk of premature sticking of the faecal collection bag. However, even when using this improved device premature sticking of the faecal collection bag to be applied has been found to occur.

Hence, there still exists a need for an applicator for a human waste collection bag which allows for easy and correct positioning of the human waste collection bag onto the desired area of the wearer by the caretaker or wearer themselves without causing discomfort to the wearer or damage to the device.

In attempting to overcome all the aforementioned problems relating to the prior art, it has now been found that a specifically designed applicator which can be utilised with adhesive faecal collection bags greatly facilitates the correct placement of the device. Furthermore such an applicator maintains the bag of the device in a folded configuration prior to the use of the device and supports the hygienic and protective packaging of the device.

### Summary of the invention

The present invention relates to human waste collection bags for babies, children or adults to be attached to a wearer. More particularly it relates to an applicator for such collection bags, which can be used to place the collection bag in the perianal or uro-genital area of a wearer and allows convenient and accurate placement. Claimed and described is an applicator (40) comprising at least one handling portion (42) and at least one contact area (44); which upon application of force between said at least one handling portion (42) and the at least one contact area (44) expands in at least one direction of expansion.

### Brief description of the drawings

It is believed that the invention will be better understood from the foregoing description in conjunction with the accompanying drawings in which:
Figure 1 is a perspective view of a preferred embodiment of a faecal collection bag .
Figure 2 is a perspective view of a urine collection bag.
Figure 3 is a side view of a preferred applicator for a faecal collection bag according to the present invention, the applicator being in its relaxed shape.
Figure 4 is a perspective view of a preferred applicator for a faecal collection bag shown in Figure 3, the applicator being in its expanded shape.
Figure 5 is a cross sectional view of the applicator of Figures 3 and 4, the dotted lines indicating the expanded shape of the applicator.
Figure 6 is a perspective view onto another preferred embodiment of an applicator for a faecal collection bag according to the present invention.

### Detailed description of the invention

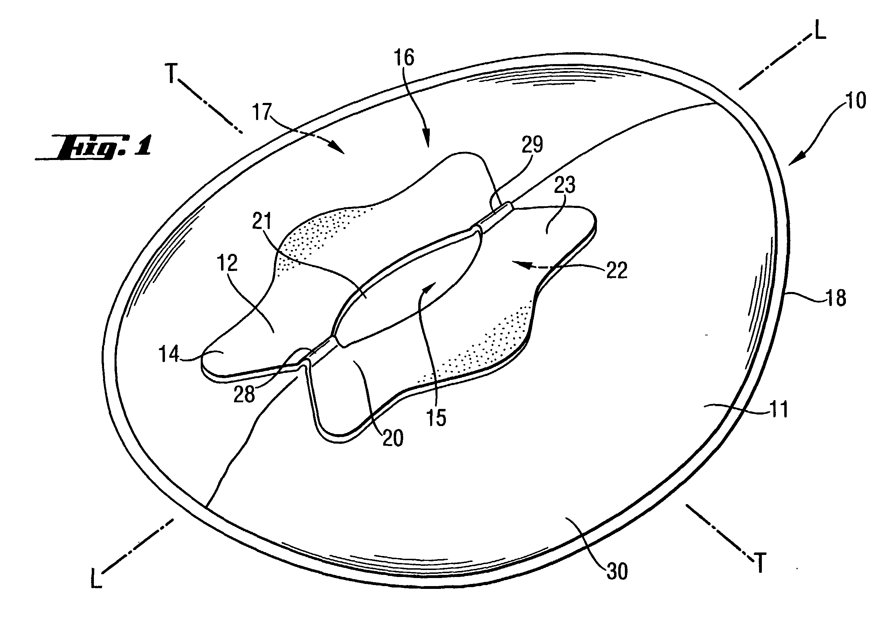

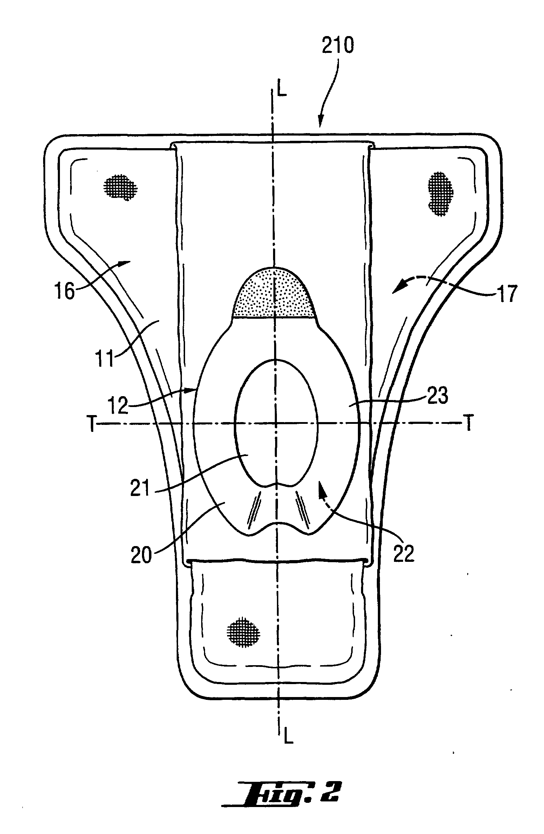

Human waste collection bags comprise faecal collection bags (10) and urine collection bags (210). Both types of collection bags can comprise similar or alike components and materials and are therefore described together. A typical faecal collection bag (10) is shown in Figure 1 and a typical urine collection bag (210) is shown in Figure 2. Faecal collection bags (10) are designed for attachment to the anal area and mainly used for collecting faeces, whereas urine collection bags (210) are attached to the urinary duct and mainly used for collecting urine. All of the above human waste collection bags are preferably designed for single use and disposal thereafter.

Typically human waste collection bags comprise a bag (11) having an aperture (21) and a flange (12) surrounding the aperture for preferably adhesive attachment to the perianal area of a wearer as visible from Figure 1. Any human waste collection bag known in the art can be provided according to the present invention.

The bag (11) as used herein is a flexible receptacle for the containment of excreted faecal matter or urine. The bag (11) is designed to safely contain any entrapped material, typically it will be liquid impermeable, yet it may be breathable. The bag (11) is designed of sufficient strength to withstand rupture in use, also when pressure on the bag (11) is exerted in typical wearing conditions, such as sitting.

The bag (11) material can comprise one or multiple layers, preferably two or three layers. The layer on the inside of the bag (11), which will typically at least partially come in contact with faecal material or urine is called the inner layer. The outermost layer of the bag (11), which will typically at least partially come in contact with the skin to the wearer and the garments of the wearer, is called the outer layer.

The layers of the bag (11) material may comprise any material, preferably so that the bag (11) is liquid impervious. The layers may in particular comprise any material such as non-wovens or films. In a preferred embodiment of the present invention a laminate may be formed from a non-woven layer and a film. The laminate can be formed by means known to the man skilled in the art.

Any non-woven layer can comprise felt fabrics, spunlaced fabrics, fluid jet entangled fabrics, air-laid fabrics, wet-laid fabrics, dry-laid fabrics, melt-blown fabrics, staple fibre carding fabrics, spunbonded fabrics, stitch-bonded fabrics, apertured fabrics, combinations of the above or the like.

Suitable film materials for any of said layers preferably comprise a thermoplastic material. The thermoplastic material can be selected from among all types of hot-melt adhesives, polyolefins especially polyethylene, polypropylene, amorphous polyolefins, and the like; material containing meltable components comprising fibres or polymeric binders including natural fibres such as cellulose - wood pulp, cotton, jute, hemp; synthetic fibres such as fibreglass, rayon, polyester, polyolefin, acrylic, polyamid, aramid, polytetrafluroethylene metal, polyimide; binders such as bicomponent high melt/low melt polymer, copolymer polyester, polyvinyl chloride, polyvinyl acetate/chloride copolymer, copolymer polyamide, materials comprising blends wherein some of the constituent materials are not meltable; air and vapour permeable materials including microporous films such as those supplied by EXXON Chemical Co., III, US under the designation EXXAIRE or those supplied by Mitsui Toatsu Co., Japan under the designation ESPOIR NO; and monolithic breathable materials such as Hytrel™ available from DuPont and Pebax™ available from ELF Atochem, France.

In a preferred embodiment a film, which is comprised in any layer, is preferably permeable to gases such as air and to vapour such as water vapour in order to avoid the problem of entrapment and condensation of moisture vapour given off by the body of the wearer and thus, the hot, clammy and uncomfortable conditions after a short period of use.

The outer layer of the bag (11) material may comprise a non-woven layer. Such material layers present an uneven surface to the skin of the wearer and thus reduce significantly the problem of occlusion and greatly improve skin healthiness.

In one preferred embodiment of the present invention the bag (11) material comprises two layers. Preferably the outer layer comprises a non-woven layer and the inner layer comprises a film.

In yet another preferred embodiment of the present invention, the bag (11) material comprises three layers, preferably one film and two non-woven layers. In an even more preferable embodiment the film is interposed between the two non-woven layers. This sequence of layers results in a closed fibrous structure, which has a particularly pleasing sensation on contact with the skin of the wearer. In yet another preferred embodiment the inner layer comprises a film and the other two layers comprise non-wovens.

The non-woven layer or the non-woven layers comprised by the bag (11) material may be hydrophobic or hydrophilic. If the bag (11) material does not comprise a film layer, preferably at least one non-woven layer is hydrophobic. As a consequence, fluid penetration is resisted through the wearer facing portion (16) and the garment facing portion (17) of the human waste collection bag. As used herein the wearer facing portion (16) is to be understood as the portion of the bag (11) generally facing towards the wearer and the garment facing portion (17) is to be understood as the portion of the bag (11) generally facing the garment of the wearer. If the bag (11) material comprises a film or a hydrophobic non-woven layer, further non-woven layers may be hydrophilic.

Typically, the non-woven layer is treated with a surface active material, such as a fluorchemical or other hydrophobic finishings, to provide the requisite hydrophobicity. The non-woven layer, however, may equally be treated with coatings of liquid impervious materials such as hot-melt adhesives or coatings of silicone or other hydrophobic compounds such as rubbers and vegetable and mineral waxes or it may be physically treated using nano-particulates or plasma coating techniques, for example.

The non-woven layer can also be treated with agents to improve the tactile perceivable softness of the wearer facing portion (16) and the garment facing portion (17). The agents include but are not limited to vegetable, animal or synthetic oils, silicone oils and the like. The presence of these agents are known to impart a silky or flannel-like feel to the non-woven layer without rendering it greasy or oily to the tactile sense of the wearer. Additionally, surfactant material, including anionic, cationic, non-ionic and amphoteric surfactants, may be added to further enhance softness and surface smoothness.

Furthermore, the non-woven layer may be impregnated with a lotion to provide desirable therapeutic or protective coating lotion benefits. The lotion coating on the wearer facing portion (16) and the garment facing portion (17) is transferable to the skin of the wearer by normal contact and wearer motion and/or body heat. Generally, mineral oil in the form of a lotion is recognised as being effective in imparting a soothing, protective coating to the skin of the wearer. It is also possible to impregnate the non-woven layer with a solid oil phase of cream formulation or to incorporate into the non-woven layer an array of pressure- or thermal- or hydrorupturable capsules containing for example, baby oil.

Depending on the shape of the bag (11) required, the bag (11) may be provided from a unitary piece of material or a number of separate pieces of material, which may be identical or different and which are sealed at their respective peripheries. The preferred shape of the bag (11) depends in particular on the intended use thereof, i.e. whether the device is intended for bedridden patients or active patients suffering from incontinence or requiring an artificial bowel or for infants.

The bag (11) described herein preferably have a wearer facing portion (16) and a garment facing portion (17), which both comprise separate pieces of material. The wearer facing portion (16) and the garment facing portion (17) are sealed at the periphery of the bag (11), thus creating a bag peripheral rim (18). The wearer facing portion (16) and the garment facing portion (17) may each independently comprise more than one section of material. Preferably the garment facing portion (17) comprises only one section of material; most preferably also the wearer facing portion (16) comprises only one section of material.

The wearer facing portion (16), the garment facing portion (17) and the pieces of material comprised by either of these portions are secured to each other by means known to the man skilled in the art, such as adhesive, thermobonding or pressure bonding in order to provide the desired bag configuration. The rim (18), at which the wearer facing portion (16) and the garment facing portion (17) are sealed together, may be provided inside the bag (11) rather than outside the bag (11), thus being coextensive with the inner surface (15) of the bag (11) rather than with the outer surface (30) of the bag (11).

Hence a variety of shapes of the bag (11) is within the scope of the present invention. Particularly, preferred shapes are flat circular type bags, cone shaped bags, truncated cone shaped bags and pyramidal or truncated pyramidal shaped bags and flat T shaped bags. For faecal collection bags (10) the truncated cone shape is most preferred, whereas for urine collection bags the flat T-shape is most preferred.

In one embodiment of the present invention the bag (11) may contain absorbent material. The absorbent material may comprise any absorbent material which is capable of absorbing and retaining liquids. The absorbent material may comprise a wide variety of liquid-absorbent materials commonly used in disposable diapers and other absorbent articles such as comminuted wood pulp, which is generally referred to as airfelt. Examples of other suitable absorbent materials include creped cellulose wadding; meltblown polymers, including coform; chemically stiffened, modified or cross-linked cellulosic fibers; tissue, including tissue wraps and tissue laminates; absorbent foams; absorbent sponges; superabsorbent polymers; absorbent gelling materials; or any other known absorbent material or combinations of materials.

The absorbent material may be positioned in the bag (11) in any suitable manner. For example, the absorbent material may be loosely arranged within the bag (11) or may be secured to the inner surface (15) of the bag (11). Any known techniques for securing absorbent material to nonwoven and film substrates may be used to secure the absorbent material to the inner surface (15) of the bag (11). The absorbent material may also be arranged to have any desired shape or configuration (e.g., rectangular, oval, circular, etc.).

The bag (11) is provided with an aperture (21) whereby faecal matter or urine is received from the body prior to storage within the bag (11) cavity. The aperture (21) is surrounded by a flange (12) and may be provided in any shape or size, such as circular, oblong, heart shaped and may be symmetrical or asymmetrical, preferably the aperture has an oblong configuration either in the longitudinal or in the transversal direction.

The flange (12) is attached to the bag (11) according to any means known to the man skilled in the art which may provide permanent or releasable attachment.

Preferably however, the flange is attached to the bag (11) by adhesive. Typically, the bag (11) will be attached to the flange, towards the outer periphery of flange so as not to cause any obstruction for the entering faecal matter or urine.

The flange may be provided in any size depending on the wearer group for which the device is intended. Similarly the flange may be provided in any shape and preferably has a symmetrical shape preferably comprising a plurality of lobes (14).

The flange comprises a garment facing portion (22) and a wearer facing portion (23). In an preferred embodiment these are two large, substantially flat surfaces, however, the flange (12) may also comprise projections, a front projection (28) and/or a rear projection (29), in case of a faecal collection bag (10) designed to fit the perineal and/or coccygeal area of the wearer and in case of a urine collection bag designed to fit the genital and/or perineal area.

The flange (12) should be made of soft, flexible and malleable material to allow easy placement of the flange (12) to the perianal or uro-genital area. Typical materials include nonwoven materials, wovens, open celled thermoplastic foams, closed-cell thermoplastic foams, composites of open celled foams and stretch nonwoven, and films. A closed-cell foam of polyethylene has been found effective, but more preferably an open celled polyurethane foam is used. Preferably, such foams have a thickness within the general range of 0.1 to 5 millimetres and a density of 5 to 250 g/l, more preferably 50 g/l. Other thermoplastic foam materials, or other suitable plastics sheet materials having the described properties of such foams (i.e., softness, pliability, stretchability, and contractability) might also be used. Preferably, the material of garment facing portion (22) of the flange (12) may extend into the defined aperture area so as to form a skirt or flap of material which prevents unintentional adhesion of the surface edges of the flange (12) defining the aperture (21) to one another during use.

The human waste collection bag further comprises an attachment means to secure the device to the wearer. Such means include straps and more preferably comprises a body-compatible pressure sensitive adhesive (20) applied to the wearer facing portion (23) of the flange (12).

The adhesive (20) is preferably covered with a release means (not shown) in order to protect the adhesive (20), such as siliconised paper. The adhesive (20) can cover the entire wearer facing portion (23) of the flange (12), more preferably the flange (12) has at least one, preferably two to six non-adhesive portions. These portions may be adhesive free or may contain inactivated or covered adhesives. As is evident from Figure 1, the adhesive is in one preferred embodiment not applied to the entire wearer facing portion (23) of the flange (12), so as to provide lobes (14) on either side of the flange (12) which are non-adhesive and can thereby serve to facilitate placement and removal of the device whilst avoiding contact with the adhesive. These lobes (14) are however preferably also covered by the release means. Before application of the human waste collection bag to the skin of the wearer, the release means if present is removed.

Any medically approved water resistant pressure sensitive adhesive may be used to attach the device to the perianal or uro-genital area of the wearer, such as hydrocolloid adhesives and hydrogel adhesives. Particularly effective adhesives in providing the desired adhesive properties to secure the flange to the skin of the wearer at the sensitive perianal area, whilst allowing for relatively painless application and removal, are formed from crosslinking polymers with a plastisicer to form a 3-dimensional matrix.

The adhesive (20) can be applied to the wearer facing portion (23) of the flange (12) by any means known in the art such as slot coating, spiral, or bead application or printing. Typically the adhesive (20) is applied at a basis weight of from 20g/m² to 2500g/m², more preferably from 500g/m² to 2000g/m² most preferably from 700g/m² to 1500g/m² depending on the end use envisioned. For example, for human waste collection bags to be used for babies the amount of adhesive (20) may be less than for human waste collection bags designed for active adult incontinence sufferers.

### Detailed description of the applicator

To allow a more detailed and clear description of the present invention, in the following paragraphs firstly a number of terms, as used herein, will be defined.

Regarding the human waste collection bag the longitudinal direction is to be understood as follows: the plane which bisects an upright standing wearer in a left and a right half shall comprise the longitudinal direction of the human waste collection bag. If a human waste collection bag comprises one or two projections (28) and/or (29) designed to fit the perineal or coccygeal area of the wearer, the longitudinal axis typically intersects these projections. The longitudinal axis is typically also an axis of symmetry of the bag (11).

The transversal axis is an axis perpendicular to the plane which bisects an upright standing wearer in a left and a right half, the axis crossing the centre of the aperture (21). The bag (11) is typically not symmetrical to the transversal axis.

Longitudinal, when used for the applicator (40), shall denote the direction which is substantially parallel to the longitudinal direction of the human waste collection bag when the applicator (40) is used for placing the human waste collection bag.

Typically applicator (40) comprises a plane of symmetry which comprises the longitudinal direction.

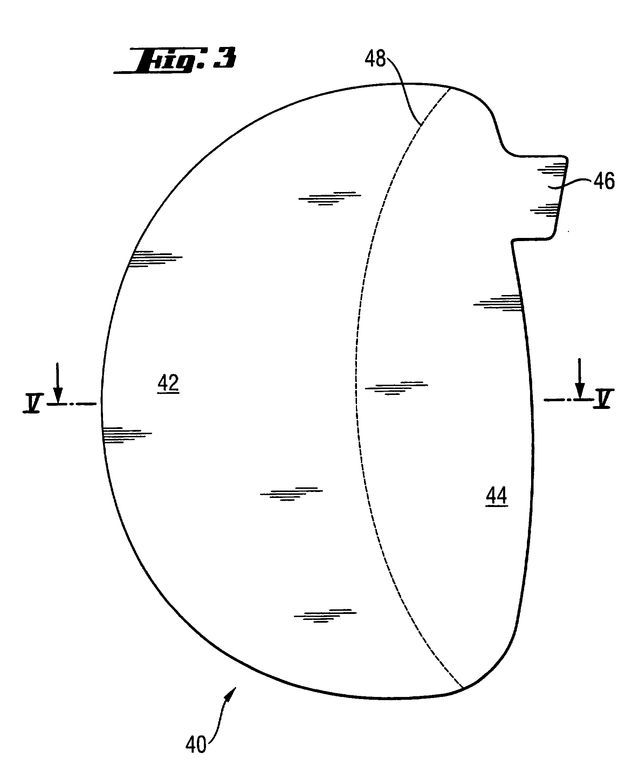

The invention relates to an applicator (40) to be used for the placing of a human waste collection bag to the uro-genital or perianal area of a wearer, preferred applicators (40) are shown in Figures 3 to 6.

The applicator (40) is typically used by a person, whether the wearer or a caretaker, and typically handled by that person using the hands. Thus, to be held and to be used to exert pressure by the caretaker or wearer, the applicator typically comprises a handling portion (42). The handling portion (42) may comprise one or more pieces of material, may be joined to the applicator (40) or may be an integral part of the applicator (40).

The applicator (40) further comprises a contact area (44), which is the area which contacts the human waste collection bag when the applicator (40) is handled using the handling portion (42). The contact area (44) may comprise one or more pieces of material, may be joined to the applicator (40) or may be an integral part of the applicator (40).

Figures 3 to 5 show a particularly preferred embodiment of the present invention which is optimal or use with a faecal collection bag. The applicator (40) depicted there comprises two essentially oval pieces of cardboard (40a, 40b) which have mirror symmetry. The handling portion (42) and the contact area (44) of the applicator (40) both comprise portions of both pieces (40a, 40b). A pivot line (48) separates the handling portion (42) and the contact area (44).

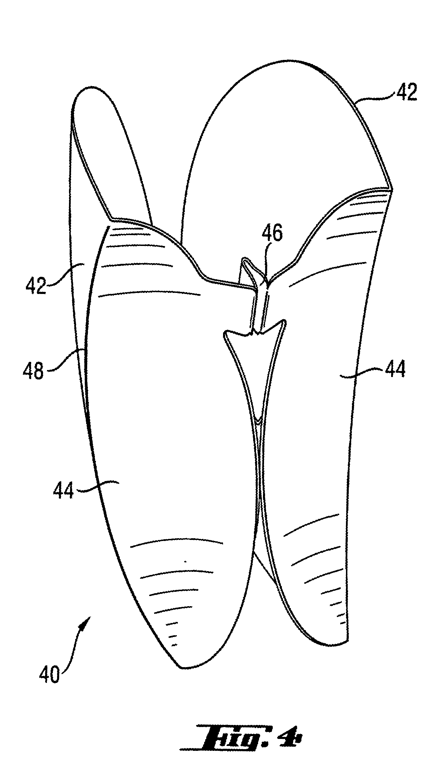

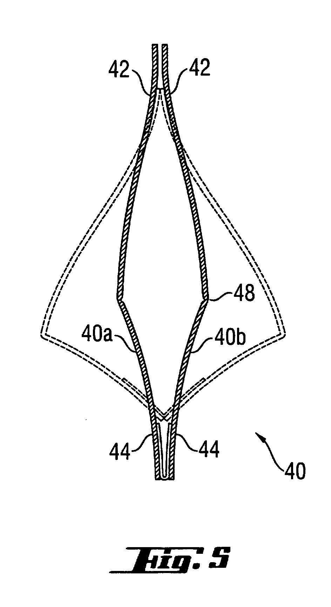

The pivot line (48) allows for the controlled change of shape and associated expansion when the applicator is used as intended, so that force is applied between said handling portion (42) and said contact area (44). Upon application of such force the applicator (40) will change from a released shape as depicted in Figure 3 to an expanded shaped as depicted in Figure 4; a cross sectional view of the two respective shapes is further given in Figure 5.

For an applicator (40) according to the present invention this expansion occurs in at least one direction, preferably this expansion occurs in a plane perpendicular to the direction of the force applied between the handling portion (42) and the contact area (44). The applicator (40) shown in Figures 3 - 5 is preferably used so that the plane of mirror symmetry defined by the pieces (40a) and (40b) of the applicator (40) and thereby the longitudinal direction of the applicator (40) as defined herein is aligned with the anal groove. When the applicator (40) is used in this position the expansion occurs in the transversal direction and hence helps in spreading apart the buttocks of a wearer when the human waste collection bag is applied.

The dimensions of the applicator (40) when measured in at least one direction, herein referred to as direction of expansion, will be larger in the expanded shape than in the relaxed shape. Preferably the dimension of the applicator (40) measured in the at least one direction of expansion will increase as compared to the dimension of the applicator (40) in the relaxed shape in the same direction preferably by at least 10%, more preferably by at least 25%, yet more preferably by at least 50%, yet even more preferably by 75 %, 100% or 200% and most preferably by at least 300%.

A further benefit of the change in shape of the applicator (40) occurring with the described expansion is the associated change in shape of the contact area (44). In the expanded state, as visible from Figures 4 and 5, the contact area has a highly body-conforming shape and is in an ideal body conforming orientation essentially vertical to the direction in which force to apply a human waste collection bag is exerted.

The body conforming shape of the contact area in the expanded state is to a large extend due to the curved contour of the pivot line (48). A curved contour of the pivot line (48) is highly preferred according to the present invention and most preferably the line is concave when seen from the contact area (44). Preferably the curvature is chosen in view of the anatomy of the intended wearer group of which the applicator (40) is to be used. The pivot line (48) shown in Figures 3 and 4 has been found optimal for an applicator (40) for a faecal collection bag to be used for a baby.

However, any other pivot line (48) is within the scope of the present invention. Moreover, any means allowing for the expansion of the applicator (40) upon application of force is within the scope of the present invention. Such means include any pivotal joint and any flexible means, for example a flexible insert, such as a rubber insert or a line of reduced flexure resistance, such as a line of perforation. Such line may be continuous or discontinuous.

In addition to its benefits for convenient and reliable application of a human waste collection bag the utilisation of the applicator (40) according to the present invention also greatly assists in assuring that the bag (11), which is preferably provided in a folded configuration prior to the placing of the human waste collection bag, remains in that folded configuration during application. The applicator (40) was also found to provide hygienic and mechanical protection for the human waste collection bag. Furthermore the applicator (40) was found to assist in the economic, aesthetically pleasing and protective packaging of the human waste collection bag.

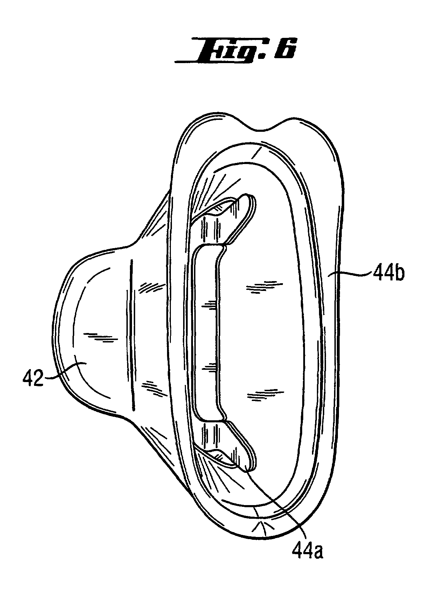

A preferred embodiment of the present invention, which is optimised in respect of the aethestic appearance of the applicator (40), is shown in Figure 6. This preferred embodiment is made of a foam material, preferred are open cell or closed cell thermoplastic foams, e.g. of polyethylene or polyurethane, but any other flexible material listed hereinafter is also suitable. The applicator (40) is so shaped that it provides a housing for the folded human waste collection bag thereby a allowing a cost effective, hygienic and discrete packaging of the device. In this embodiment the applicator (40) comprises two contact areas: the pegs (44a) and the rim (44b). Upon application of pressure onto the handling portion (42) of the applicator (40) the rim (44b) will expand, typically mostly in the transversal direction, and thereby engage in a spreading action, which in case of a faecal collection bag will lead to the spreading of the buttocks of the wearer. The pegs (44a) are designed to apply pressure onto the coccygeal and perineal areas, where the application of pressure is particularly important of a good sealing when an adhesive flange (12) is used.

This preferred embodiment does not comprise a pivot line (48) but due to its shape and the flexible material used, the described expansion and spreading action occurs when the rim (44b) has contact with the body of a wearer and forces is exerted via the handling portion (42).

The applicator (40) depicted in Figure 6 is further an example of an applicator (40) comprising a cavity for storing the human waste collection bag prior to use. This cavity is provided inside the handling portion (42) and offers sufficient space to store the human waste collection bag, in particular when it is folded prior to use. This cavity can be closed and sealed against air, water, dirt or the like by covering the opening which is surrounded by the rim (44b) with a sheet, e.g. from polyethylene, polypropylene, paper or the like. Preferably such sheet is releasably attached to the rim (44b), e.g. by using a suitable adhesive. In such preferred embodiment the applicator (40) serves as an aesthetically pleasing and functional package for the human waste collection bag.

An applicator (40) according to a present invention can be made in a variety of shapes and from a variety of materials. The applicator (40) may be made from one unitary piece of material or may comprise several pieces of material.

The material should allow the transfer of the required forces from the handling portion (42) of the applicator (40) to the contact area (44). Preferably the material is inexpensive and suitable for a mass manufacturing process. Preferably the material is environmentally friendly for disposal, in particular for flushing in a toilet.

Preferred materials are wood, metal, plastic and cardboard. Cardboard maybe be provided with a coating, e.g. a wax coating to improve the hygienic handling of the applicator (40) and to prevent any dirt from easily sticking to or otherwise soiling the applicator (40). Preferably the cardboard has a thickness from 0.01 to 5 mm, more preferably from 0.1 to 1 mm, most preferably from 0.3 to 0.7 mm.

Preferred materials also include those used for tampon applicators, which are typically provided from plastics, polymers or flushable smooth surfaced cardboard. Materials such as paper or wood pulp which are also used for tampon applicators are hence also within the scope of the present invention. More preferred materials are disclosed in EP 613672 A1, where a commercially effective and aesthetically-acceptable rapidly-disintegrating water-soluble disposable tampon applicator and a method for making it are disclosed. The materials disclosed in EP 613672 A1 such as water-soluble polymers having a coating comprising a water-insoluble polymeric material selected from the group consisting of wax (comprising natural wax and synthetic wax), hydrogenated vegetable oil, and food grade shellac, may also be utilised for the application herein. Among the preferred water-soluble polymers is polyvinyl alcohol and thermoplastic starch.

The applicator (40) may further comprise various labels, e.g. colour labels. One such label can be used to indicate which portion of the applicator is to be brought in registry with a particular portion of the human waste collection bag. For example, a colour label may indicate which portion of the applicator (40) is to be brought in registry with the centre of the aperture (21).

Similarly colour labels on the applicator (40) can be also used to indicate how the applicator in combination with the human waste collection bag is to be oriented for placement, e.g. with regard to the perineal and the coccygeal areas of a wearer. Furthermore the applicator (40) can be labelled to provide usage instructions or any other written instructions.

In a preferred embodiment the human waste collection bag is provided in a particular configuration prior to use. In that configuration, the flange (12) is folded along the longitudinal axis to allow easier placement of the flange (12) in-between the buttocks of a wearer. Furthermore the bag (11) is preferably folded, which provides numerous advantages. For example, folding of the bag (11) allows a smaller packaging format of the human waste collection bag, thus reducing the costs for transport and packaging material. Furthermore, the handling of the human waste collection bag is more convenient if the bag (11) is folded, since the bag (11) may otherwise cover parts of the applicator (40) e.g. the handling portion (44). The bag (11), when not folded, may also cover parts of the body of the wearer, so that the person placing the human waste collection bag cannot sufficiently visually control the placing.

Most preferably the applicator (40) and the human waste collection bag in combination are provided in a particular configuration prior to use. This allows ready application of the human waste collection bag with only a few handling steps, since there is no need to have separate items at hand (the applicator (40) and the human waste collection bag) and to position the applicator on the appropriate areas of the human waste collection bag for application.

The applicator (40) may be provided with a means to hold the applicator (40) and the human waste collection bag together. This means will typically also ensure the correct positioning of the applicator (40) relative to the human waste collection bag and help to maintain the folded configuration of the bag (11), if the bag is folded. Such a means preferably are provided in the form of any string or band, which may be provided in form of a closed loop, such as a rubber band. Such a means may also be provided in form of a clamp or a clip, made from any suitable material such as plastic or metal.

The use of an applicator (40) for the placing of a human waste collection bag according to the present invention preferably comprises the following steps:
a) unpacking of the human waste collection bag and the applicator (40);
b) removing the release means from the adhesive (20) provided on said flange (12), preferably using one hand for the removal of the release means while holding the human waste collection bag and the applicator (40) with the other hand;
c) supporting the placement body position of the wearer, using one hand, e.g. for a faecal collection bag (10) to be used on a baby holding up a babies legs, while holding the human waste collection bag and the applicator (40) with the other hand;
d) placing said human waste collection bag in the perianal area or uro-genital area of the wearer preferably, when the human waste collection bag is releasably attached to the applicator (40), by using the handling portion (42) of the applicator (40);
e) letting said adhesive (20) on said flange (12) attach to the body of the wearer;
f) exerting pressure towards the perineal area of the wearer, the coccygeal area of the wearer, the uro-genital area or/and other areas of the wearer to be in contact with the adhesive (20), by using the applicator (40), either at the same time or in any sequence;
g) by using the applicator spreading the buttocks of a wearer - typically when using the applicator (40) to apply a faecal collection bag (10) to the perianal area of a wearer - and/or spreading the thighs of a wearer - typically when using the applicator (40) to apply a urine collection bag (210) to the uro-genital area of a wearer;
h) separating of the applicator (40) from the human waste collection bag while holding it using the handling portion (42) , e.g. by pulling in a direction substantially away from the perianal area of the wearer;
i) unfolding the bag (11).

Depending on the used embodiment of the present invention and conditions of the placement numerous other handling steps in placing the human waste collection bag using an applicator (40) may also be undertaken, different handling steps may be undertaken or certain of the mentions handling steps may not be involved.

## Claims

1. An applicator (40), to be used for the placement of a human waste collection bag on a wearer, said applicator (40) comprising at least one handling portion (42) and at least one contact area (44); **characterised in that**
said applicator (40) upon application of force between said handling portion (42) and said at least one contact area (44) expands in at least one direction of expansion.

2. An applicator (40) according to Claim 1 **characterised in that** said at least one direction of expansion lies in a plane perpendicular to the direction of said force.

3. An applicator (40) according to any one of the preceding claims, **characterised in that** said at least one direction of expansion is oriented in the transversal direction as defined herein.

4. An applicator (40) according to any one of the preceding claims, said applicator (40) having a relaxed shape and an expanded shape and a first dimension measured in said direction of expansion in said relaxed shape and a second dimension measured in said direction of expansion in said expanded shape, **characterised in that** second dimension is at least 10 % greater than said first dimension.

5. An applicator (40) according to any one of the preceding claims, said applicator (40) being provided with a cavity to store a human waste collection bag prior to use.

6. Use of an applicator (40) according to any one of the preceding claims for the placement of a faecal collection bag (10) onto a wearer, **characterised in that** said applicator (40) is further used to spread the buttocks of said wearer.

7. Use of an applicator (40) according to any one of the preceding claims for the placement of a urine collection bag (210) onto a wearer, **characterised in that** said applicator (40) is further used to spread the thighs of said wearer.
